## Europäisches Patentamt
## European Patent Office
## Office européen des brevets

(19)

(11) Veröffentlichungsnummer: **0 033 518**
**B1**

(12) **EUROPÄISCHE PATENTSCHRIFT**

(45) Veröffentlichungstag der Patentschrift:
**25.05.83**

(51) Int. Cl.³: **C 07 D 501/20, A 61 K 31/545**

(21) Anmeldenummer: **81100643.6**

(22) Anmeldetag: **29.01.81**

(54) **Kristalline Salze eines Cephalosporins, ihre Herstellung und sie enthaltende pharmazeutische Zusammensetzungen.**

(30) Priorität: **01.02.80 CH 836/80**

(43) Veröffentlichungstag der Anmeldung:
**12.08.81 Patentblatt 81/32**

(45) Bekanntmachung des Hinweises auf die Patenterteilung:
**25.05.83 Patentblatt 83/21**

(84) Benannte Vertragsstaaten:
**AT BE CH DE FR GB IT LI LU NL SE**

(56) Entgegenhaltungen:
**EP-A-0 001 125**
**EP-A-0 008 343**

(73) Patentinhaber: **CIBA-GEIGY AG, Patentabteilung Postfach, CH-4002 Basel (CH)**

(72) Erfinder: **Scartazzini, Riccardo, Dr., Conrad Ferdinand Meyer-Strasse 38, CH-4059 Basel (CH)**

(74) Vertreter: **Zumstein, Fritz sen., Dr. et al, Bräuhausstrasse 4, D-8000 München 2 (DE)**

## Kristalline Salze eines Cephalosporins, ihre Herstellung und sie enthaltende pharmazeutische Zusammensetzungen

Die Erfindung betrifft neue Salze, insbesondere das kristalline Hydrochlorid und das kristalline Hydrobromid des $7\beta$-[2-(2-Amino-4-thiazolyl)-2-methoxyiminoacetamido]-3-cephem-4-carbonsäure-pivaloyloxymethylesters, Verfahren zu ihrer Herstellung, sowie pharmazeutische Präparate enthaltend diese Verbindungen.

Der $7\beta$-[2-(2-Amino-4-thiazolyl)-2-methoxyiminoacetamido]-3-cephem-4-carbonsäure-pivaloyloxymethylester ist beispielsweise aus der Europäischen Patentanmeldung Nr. 8 343 bekannt. Diese Verbindung und ihre Salze, insbesondere die obengenannten, sind wertvolle, antibiotisch wirksame Substanzen, die insbesondere als antibakterielle Antibiotika verwendet werden können.

Der Pivaloyloxymethylester, sowohl als freie Base als auch als Hydrochlorid oder Hydrobromid, hat gegenüber dem ebenfalls bekannten Natriumsalz der $7\beta$-[2-(2-Amino-4-thiazolyl)-2-methoxyiminoacet-amido]-3-cephem-4-carbonsäure den Vorteil, daß er an Versuchstieren, z. B. an der Ratte, nach p. O. Applikation signifikant besser aus dem Magen-Darm-Trakt resorbiert wird und deshalb in chemotherapeutischen Versuchen oral besser wirksam ist. Beispielsweise hat der Pivaloyloxymethylester als freie Base bei zweimaliger oraler Applikation an der Maus gegen grampositive Kokken, wie Staphylococcus aureus 10B und Streptococcus pyogenes aronson, eine $ED_{50}$ von etwa 0,7 bis 30 mg/kg, gegen Enterobakterien, z. B. Escherichia coli 205, Escherichia coli 2018, Escherichia coli 205 $R^+_{TEM}$, Klebsiella pneumoniae 327, Proteus mirabilis 774, Salmonella Stanley und Proteus morganii 2359, eine $ED_{50}$ von etwa $<0,1$ bis 12 mg/kg, und gegen Pseudomonas sp., wie Pseudomonas aeruginosa, eine $ED_{50}$ von $<100$ mg/kg. Die neuen Salze des Esters werden im Vergleich zur freien Base vollständiger resorbiert und weisen dementsprechend in einigen Fällen, das Hydrochlorid z. B. gegen Streptococcus pyogenes Aronson, Escherichia coli 205 und 218, Klebsiella pneumonia 327, Proteus morganii 2359 und Pseudomonas aeruginosa ATCC 12 055, eine höhere chemotherapeutische Wirksamkeit auf.

Einer der Nachteile des Pivaloyloxymethylesters ist, daß er schwer zu reinigen ist. So ist es noch nicht gelungen, ihn in kristalliner Form zu fassen. Wegen seines amorphen und unreinen Charakters besitzt er eine geringe Stabilität, die sich bei der Lagerung und bei der Pressung von Tabletten nachteilig auswirkt. Bei der Verarbeitung zu Tabletten und beim Abfüllen in Ampullen macht sich ferner seine relativ geringe Rieselfähigkeit unangenehm bemerkbar. Die geringe Wasserlöslichkeit des Esters bewirkt eine uneinheitliche Resorption nach oraler Applikation.

Der bisher nur in Form der freien Base bekannte Pivaloyloxymethylester hat also gewisse Eigenschaften, die für ein Arzneimittel unerwünscht sind, da sie die Herstellung und Anwendung von daraus bereiteten pharmazeutischen Darreichungsformen erschweren. Es bestand demnach ein Bedürfnis nach Derivaten, die für die genannten Zwecke geeigneter sind.

Auf der Suche nach geeigneten Derivaten wurden nun überraschenderweise das Hydrochlorid und das Hydrobromid des $7\beta$-[2-(2-Amino-4-thiazolyl)-2-methoxyiminoacetamido)-3-cephem-4-carbonsäure-pivaloyloxymethylesters in kristalliner Form gefunden, die die an ein Arzneimittel zu stellenden Eigenschaften in wesentlich besserem Maße besitzen.

Diese Salze lassen sich, aufgrund ihrer Kristallisationsfähigkeit viel einfacher und besser reinigen als die freie Base. Weitere Vorteile sind ihre erhöhte Stabilität und Lagerfähigkeit, und ihre bessere Verarbeitung zu pharmazeutischen Darreichungsformen. Insbesondere lassen sie sich leichter trocknen und, wegen ihrer erhöhten Stabilität bei höheren Temperaturen und Drucken, auch besser zu Tabletten pressen. Aufgrund einer größeren Rieselfähigkeit lassen sie sich leichter in Kapseln oder Vials abfüllen. Die erhöhte Wasserlöslichkeit bewirkt im Magen-Darm-Trakt eine einheitlichere Verteilung und eine vollständigere Resorption. Gegenüber der freien Base sind folglich, insbesondere bei oraler Applikation, große Vorteile gegeben.

Die erfindungsgemäßen Salze werden auf an sich bekannte Weise hergestellt. Das Verfahren zur Herstellung von kristallinem $7\beta$-[2-(2-Amino-4-thiazolyl)-2-methoxyiminoacetamido]-3-cephem-4-carbonsäure-pivaloyloxymethylester-hydrochlorid oder -hydrobromid ist dadurch gekennzeichnet, daß man den $7\beta$-[2-(2-Amino-4-thiazolyl)-2-methoxyiminoacetamido]-3-cephem-4-carbonsäure-pivaloyloxymethylester durch Behandlung mit Chlorwasserstoff oder Bromwasserstoff in das Hydrochlorid oder Hydrobromid überführt und das erhaltene Salz zur Kristallisation bringt.

Die freie Base kann in roher oder gereinigter Form eingesetzt werden. Der Chlor- oder Bromwasserstoff wird in wäßriger Form oder bevorzugt in wasserfreier Form, und zwar bevorzugt in etwa äquivalenter Menge eingesetzt, d. h. in Abhängigkeit von der Reinheit der eingesetzten Base werden etwa 0,8—1,2, bevorzugt 1—1,1 Moläquivalente, an Chlor- oder Bromwasserstoff eingesetzt. Die Salzbildung wird mit Vorteil in einem Lösungsmittel, wie Wasser, einem organischen Lösungsmittel oder Mischungen davon, durchgeführt. Als organische Lösungsmittel für die Base können beispielsweise Alkohole, wie Methanol, Äthanol, n-Propanol, Isopropanol, Butanol, Isobutanol, Ketone, z. B. Aceton oder Methyläthylketon, Nitrile, z. B. Acetonitril, Äther, z. B. Tetrahydrofuran, Dioxan, Diäthyläther oder höhere Äther, Sulfoxide, z. B. Dimethylsulfoxid, Amide, z. B. Formamid, N,N-Dimethylformamid oder N,N-Dimethylacetamid, Ester, z. B. Äthylacetat, Methylcellulose, oder halogenierte Kohlenwasserstoffe, z. B. Methylenchlorid, Chloroform oder ähnliche verwendet werden. Der Chlor- oder Bromwasserstoff wird am besten ebenfalls in einem dieser Lösungsmittel gelöst und zur Lösung des Amins gefügt. Der Chlorwasserstoff kann auch gasförmig in die Lösung des Amins eingeleitet werden. Die Chlor- oder Bromwasserstofflösung kann in gesättigter oder in ungesättigter bis verdünnter Form angewendet werden. Bevorzugt wird die Base in Methylenchlorid gelöst und die berechnete Menge einer Lösung von Chlor- oder Bromwasserstoff in einem organischen Lösungsmittel, bevorzugt ebenfalls Methylenchlorid oder auch Methylenbromid oder Diäthyläther, zugefügt.

Nach Zugabe des Chlor- oder Bromwasserstoffs wird die das Salz enthaltende Lösung durch Abdampfen des Lösungsmittels konzentriert und/oder mit einem unpolaren Lösungsmittel, z. B. Diäthyläther oder einem Kohlenwasserstoff, wie Pentan oder Hexan, versetzt, worauf, nach Überschreitung des Löslichkeitsproduktes, das Hydrochlorid oder Hydrobromid ausfällt. Je nach Reinheitsgrad des verwendeten Ausgangsmaterials fallen die Salze in amorpher oder bereits in kristalliner Form aus. Durch Abkühlung der Fällungslösung kann die Fällung vervollständigt werden.

Die Salzbildung wird bei einer Temperatur zwischen etwa $-10$ und $+40°C$, bevorzugt zwischen etwa $0°$ und $30°C$ vorgenommen. Hohe Temperaturen und ein Überschuß an Chlor- oder Bromwasserstoff sind zu vermeiden, da in diesen Fällen Zersetzung eintreten kann. Das ausgefallene Salz wird durch übliche Abtrennmethoden, beispielsweise durch Filtration oder Zentrifugation, isoliert und mit einem unpolaren Lösungsmittel, in dem die Kristalle unlöslich oder schwerlöslich sind, gewaschen. Falls die Qualität des Niederschlages bezüglich Reinheit und Kristallform noch unbefriedigend ist, kann sie durch Umlösen aus einem oder mehreren der vorgenannten Lösungsmittel verbessert werden. Bevorzugt wird der erste Niederschlag des Salzes durch Umkristallisation aus Methylenchlorid gereinigt. Während die amorphen Salze, insbesondere bei Zimmertemperatur oder leichtem Erwärmen, in Methylenchlorid gut löslich sind, sind die kristallinen Formen praktisch unlöslich. Sie bilden sich beim Stehen, insbesondere bei niederen Temperaturen, etwa bei $0-5°C$. Nötigenfalls wird der Umkristallisationsprozess wiederholt bis ein analysenreines Salz erhalten wird.

Die Trocknung des Präparates erfolgt mit Vorteil im Hochvakuum bei etwa Zimmertemperatur bis etwa $30°C$, gegebenenfalls in Gegenwart eines der üblichen Trocknungsmittel.

Es ist bekannt, daß 2-Aminothiazolverbindungen der vorliegenden Art gegebenenfalls in ihrer tautomeren 2-Iminothiazolinform vorliegen können. Gemäß NMR-Spektrum liegt das erfindungsgemäße Hydrochlorid in DMSO.d6 in der 2-Ammoniothiazolform vor. In einem andern Lösungsmittel könnte aber ebenfalls die 2-Iminiothiazolform gebildet werden.

Die kristallinen Salze der vorliegenden Erfindung können zur Herstellung von pharmazeutischen Präparaten verwendet werden, die eine wirksame Menge der Aktivsubstanz gegebenenfalls im Gemisch mit anorganischen oder organischen, festen oder flüssigen, pharmazeutisch verwendbaren Trägerstoffen enthalten, die sich insbesondere zur oralen Verabreichung eignen. Zur oralen Verabreichung verwendet man Tabletten, Steckkapseln oder Gelatinekapseln, die den Wirkstoff zusammen mit Verdünnungsmitteln, z. B. Laktose, Dextrose, Sucrose, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z. B. Magnesiumaluminiumsilikat, Stärken, wie Mais-, Weizen-, Reis- oder Pfeilwurzstärke, Gelatine, Tragant, Methylcellulose, Natriumcarboxymethylcellulose und/oder Polyvinylpyrrolidon, und, wenn erwünscht, Sprengmittel, z. B. Stärken, Agar, Alginsäure oder Salze davon, wie Natriumalginat, und/oder Brausemischungen, oder Absorptionsmittel, Farbstoffe, Geschmackstoffe und Süßmittel. Suppositorien sind in erster Linie Fettemulsionen oder Suspensionen.

Die pharmazeutischen Präparate können sterilisiert sein und/oder Hilfsstoffe, z. B. Konservier-, Stabilisier-, Netz- und/oder Emulgiermittel, Löslichkeitsvermittler, und/oder Puffer enthalten. Die vorliegenden pharmazeutischen Präparate, die, wenn erwünscht, weitere phamakologisch wertvolle Stoffe enthalten können, werden in an sich bekannter Weise, z. B. mittels konventioneller Mischungs-, Lösungs-, oder Lyophilisierungsverfahren, hergestellt und enthalten von etwa 0,1%—100%, insbesondere von etwa 1%— etwa 50%, Lyophilisate bis zu 100% des Aktivstoffes. In Abhängigkeit von der Art und Schwere der Infektion und dem Zustand des infizierten Organismus verwendet man tägliche Dosen von etwa 0,5 g bis etwa 5 g p. o. zur Behandlung von Warmblütern von etwa 70 kg Gewicht.

Die folgenden Beispiele dienen zur Illustration der Erfindung; Temperaturen werden in Celsiusgraden angegeben. $R_f$-Angaben für Dünnschichtchromatographie: DS: auf Silikagel-Fertigplatten SL 254 der Firma Antec, Birsfelden.


## Beispiel 1

Zu einer auf $0°$ gekühlten Lösung von 4,97 g 7$\beta$-[2-(2-Amino-4-thiazolyl)-2-methoxyiminoacetamido]-3-cephem-4-carbonsäurepivaloyloxymethylester (freie Base) in 50 ml $CH_2Cl_2$ werden 61 ml einer 0.18M HCl-Lösung in $CH_2Cl_2$ (1.1 Moläquivalent, hergestellt durch Einleiten von trockenem gasförmigem Chlorwasserstoff in trockenes $CH_2Cl_2$) gegeben. Nach 10minütigem Rühren wird die Lösung mit Diäthyläther versetzt, wobei ein Niederschlag ausfällt. Das Gemisch wird 1/2 Stunde bei 0° nachgerührt, der Niederschlag abfiltriert, mit Diäthyläther gewaschen und im Hochvakuum bei 30° getrocknet. Man erhält so das rohe Hydrochlorid als hellbeiges Pulver, das in ca. 50 ml $CH_2Cl_2$ gelöst, etwas eingeengt und über Nacht bei etwa +5° stehen gelassen wird. Die auskristallisierte Verbindung wird abfiltriert, mit wenig $CH_2Cl_2$ und Diäthyläther gewaschen und wie zuvor getrocknet. Man erhält das farblose 7$\beta$-[2-(2-Amino-4-thiazolyl)-2-methoxyiminoacetamido]-3-cephem-4-carbonsäure-pivaloyloxymethylester-hydrochlorid vom Smp. 187—191°. $[\alpha]_D^{20} = +82 \pm 1°$ (c = 0,689% in Methanol); DS: $R_f \sim 0.29$ (Silikagel; Essigester); UV-Spektrum (EtOH): Maxima bei 235 ($\varepsilon$ = 16 600); 245 (sh) und 296 (sh)m$\mu$; IR-Spektrum (Nujol): Absorptionsbanden bei 3240; 1778; 1757; 1750; 1738; 1658; 1626; 1590; 1665 cm$^{-1}$;

NMR-Spektrum (DMSO.d6) : 100 MHz.
$\delta$ = 1.18 s, 9 H ( — C(CH₃)₃)
$\delta$ = 3.70 m, 2 H (H-2)
$\delta$ = 3.98 s, 3 H (OCH₃)
$\delta$ = 5.19 2H, d, J = 4.5 (H-6)
$\delta$ = 5.96 m, 3 H (H-7 und — OCH₂O — )
$\delta$ = 6.63 t, 1 H (H-3)
$\delta$ = 6.97 s, 1 H (Thiazol-H)
$\delta$ = 9.56 b, 3 H ( — NH₃⊕)
$\delta$ = 9.83 d, 1 H, J = 8 (CONH)

Mikroanalyse:

| | | | | | |
|---|---|---|---|---|---|
| gefunden: | C 42,79 | H 4,44 | N 13,20 | S 11,71 | Cl 6,51% |
| berechnet: | C 42,74 | H 4,53 | N 13,12 | S 12,01 | Cl 6,64% |

Röntgen-Pulver-Analyse: Die Probe ist kristallin.

### Beispiel 2

Zu einer eisgekühlten Lösung von 497 mg 7$\beta$-[2-(2-Amino-4-thiazolyl)-2-methoxyiminoacetamido]-3-cephem-4-carbonsäurepivaloyloxymethylester (freie Base) in 5 ml $CH_2Cl_2$ werden unter Rühren 2,72 ml einer 0.46 M HBr-Lösung in $CH_2Cl_2$ ( $\triangleq$ 1,25 Moläquivalente) gegeben. Nach 10minütigem Rühren wird die Lösung mit 35 ml Diäthyläther versetzt, wobei ein Niederschlag ausfällt. Das Gemisch wird 1/2 Stunde bei 0° nachgerührt, der Niederschlag abfiltriert, mit Diäthyläther gewaschen und auf der Nutsche getrocknet. Das erhaltene leicht orange Pulver wird in 2.5 ml $CH_2Cl_2$ gelöst, worauf das Produkt wieder auskristallisiert. Nach Zugabe von 5 ml $CH_2Cl_2$ wird eine Stunde unter Eiskühlung gerührt. Abfiltration, Waschen der erhaltenen Kristalle mit $CH_2Cl_2$ und Diäthyläther und Trocknen am Hochvakuum ergibt das 7$\beta$-[2-(2-Amino-4-thiazolyl)-2-methoxyiminoacetamido]-3-cephem-4-carbonsäurepivaloyloxymethylester-hydrobromid vom Smp. 215° (Zersetzung). DS: $R_f \sim 0.31$ (Silikagel : Essigester); UV-Spektrum (EtOH): Maxima bei 225 ($\varepsilon$ = 17 520); 231 ($\varepsilon$ = 16 920); 237 ($\varepsilon$ = 16 240); 243 ($\varepsilon$ = 15 600) und 249 ($\varepsilon$ = 14 420) m$\mu$; IR-Spektrum (Nujol): Absorptionsbanden bei 3245; 1776; 1757; 1750; 1738; 1658; 1628; 1590; 1570; 1560 cm$^{-1}$;

NMR-Spektrum (DMSO.d6): 100 MHz
$\delta$ = 1.18 s, 9 H ( — C(CH₃)₃)
$\delta$ = 3.70 m, 2 H (H-2)
$\delta$ = 3.99 s, 3 H (OCH₃)
$\delta$ = 5.20 d, 2 H; J = 5.0; (H-6)
$\delta$ = 5.86 m, 3 H (H-7 und — O — CH₂ — O — )
$\delta$ = 6.65 t, 1 H (H-3)
$\delta$ = 9.07 b, 3 H ( — NH₃⊕)
$\delta$ = 9.84 d, 1 H J = 8 (CONH)

### Beispiel 3

Kapseln, enthaltend 0,25 g 7$\beta$-[2-(2-Amino-4-thiazolyl)-2-methoxyiminoacetamido]-3-cephem-4-carbonsäure-pivaloyloxymethylester-hydrochlorid, werden wie folgt hergestellt:

Zusammensetzung (für 1000 Kapseln):

4

0 033 518

7β-[2-(2-Amino-4-thiazolyl)-2-methoxyiminoacetamido]-3-cephem-
4-carbonsäure-pivaloyloxymethylester-hydrochlorid · 250 000 g
Maisstärke · 50 000 g
Polyvinylpyrrolidon · 15 000 g
Magnesiumstearat · 5 000 g
Äthanol · q. s.

Das 7β-[2-(2-Amino-4-thiazolyl)-2-methoxyiminoacetamido]-3-cephem-4-carbonsäure-pivaloyloxymethylester-hydrochlorid und die Maisstärke werden vermischt und mit einer Lösung des Polyvinylpyrrolidons in 50 g Äthanol befeuchtet. Die feuchte Masse wird durch ein Sieb mit einer Maschenweite von 3 mm gedrückt und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Größe 0 abgefüllt.

## Beispiel 4

Tabletten, enthaltend 250 mg 7β-[2-(2-Amino-4-thiazolyl)-2-methoxyiminoacetamido]-3-cephem-4-carbonsäure-pivaloyloxymethylesterhydrochlorid werden wie folgt hergestellt:

Zusammensetzung (für 1 Tablette):
7β-[2-(2-Amino-4-thiazolyl)-2-methoxyiminoacetamido]-3-cephem-
4-carbonsäure-pivaloyloxymethylester-hydrochlorid · 250 mg
Mikrokristalline Cellulose · 80 mg
Natrium-carboxymethyl-stärke · 10 mg
Magnesiumstearat · 3 mg
Talk · 7 mg
350 mg

Der Wirkstoff wird mit den Zusatzstoffen homogen vermischt und zu Tabletten gepreßt.
Zur Herstellung vom Filmdragées werden die Tabletten je mit 1 mg wäßrigem Lack überzogen.
Anstelle von Natrium-carboxymethyl-stärke kann auch Natrium-carboxymethylcellulose eingesetzt werden.
Auf die gleiche Weise werden Kapseln und Tabletten mit 7β-[2-(2-Amino-4-thiazolyl)-2-methoxyimi-noacetamido]-3-cephem-4-carbonsäure-pivaloyloxymethylester-hydrobromid als Wirkstoff herge-stellt.

## Patentansprüche für die Vertragsstaaten: BE, CH, LI, DE, FR, GB, JT, LU, NL, SE

1. Das kristalline 7β-[2-(2-Amino-4-thiazolyl)-2-methoxyiminoacetamido]-3-cephem-4-carbonsäure-pivaloyloxymethylester-hydrochlorid.
2. Das kristalline 7β-[2-(2-Amino-4-thiazolyl)-2-methoxyiminoacetamido]-3-cephem-4-carbonsäure-pivaloyloxymethylester-hydrobromid.
3. Verfahren zur Herstellung von kristallinem 7β-[2-(2-Amino-4-thiazolyl)-2-methoxyiminoacetami-do]-3-cephem-4-carbonsäure-pivaloyloxymethylester-hydrochlorid oder -hydrobromid, dadurch ge-kennzeichnet, daß man den 7β-[2-(2-Amino-4-thiazolyl)-2-methoxyiminoacetamido)-3-cephem-4-carbonsäure-pivaloyloxymethylester durch Behandlung mit Chlor- oder Bromwasserstoff in das Hydrochlorid oder Hydrobromid überführt und dieses zur Kristallisation bringt.
4. Pharmazeutische Präparate enthaltend das kristalline Hydrochlorid gemäß Patentanspruch 1.
5. Pharmazeutische Präparate enthaltend das kristalline Hydrobromid gemäß Patentanspruch 2.
6. Verwendung des kristallinen Hydrochlorids gemäß Patentanspruch 1 zur Herstellung von pharmazeutischen Präparaten.
7. Verwendung des kristallinen Hydrobromids gemäß Patentanspruch 2 zur Herstellung von pharmazeutischen Präparaten.

## Patentansprüche für den Vertragsstaat AT

1. Verfahren zur Herstellung von kristallinem 7β-[2-(2-Amino-4-thiazolyl)-2-methoxyiminoacetami-do]-3-cephem-4-carbonsäure-pivaloyloxymethylester-hydrochlorid, dadurch gekennzeichnet, daß man den 7β-[2-(2-Amino-4-thiazolyl)-2-methoxyiminoacetamido]-3-cephem-4-carbonsäure-pivaloyloxymethylester durch Behandlung mit Chlorwasserstoff in das Hydrochlorid überführt und dieses zur Kristallisation bringt.
2. Verfahren zur Herstellung von kristallinem 7β-[2-(2-Amino-4-thiazolyl)-2-methoxyiminoacetami-

5

do]-3-cephem-4-carbonsäure-pivaloyloxymethylester-hydrobromid, dadurch gekennzeichnet, daß man den 7β-[2-(2-Amino-4-thiazolyl)-2-methoxyiminoacetamido]-3-cephem-4-carbonsäure-pivaloyloxymethylester durch Behandlung mit Bromwasserstoff in das Hydrobromid überführt und dieses zur Kristallisation bringt.

3. Verwendung des nach Patentanspruch 1 erhaltenen kristallinen Hydrochlorids zur Herstellung von pharmazeutischen Präparaten.

4. Verwendung des nach Patentanspruch 2 erhaltenen kristallinen Hydrobromids zur Herstellung von pharmazeutischen Präparaten.

## Claims for the Contracting States: BE, CH, DE, FR, GB, IT, LI, LU, NL, SE

1. Crystalline 7β-[2-(2-amino-4-thiazolyl)-2-methoxyiminoacetamido]-3-cephem-4-carboxylic acid pivaloyloxymethyl ester hydrochloride.

2. Crystalline 7β-[2-(2-amino-4-thiazolyl)-2-methoxyiminoacetamido]-3-cephem-4-carboxylic acid pivaloyloxymethyl ester hydrobromide.

3. Process for the production of crystalline 7β-[2-(2-amino-4-thiazolyl)-2-methoxyiminoacetamido]-3-cephem-4-carboxylic acid pivaloyloxymethyl ester hydrochloride or hydrobromide, characterised in that 7β-[2-(2-amino-4-thiazolyl)-2-methoxyiminoacetamido]-3-cephem-4-carboxylic acid pivaloyloxymethyl ester is converted by treatment with hydrogen chloride or hydrogen bromide into the hydrochloride or hydrobromide and the resulting salt is caused to crystallise.

4. Pharmaceutical preparations containing the crystalline hydrochloride according to patent claim 1.

5. Pharmaceutical preparations containing the crystalline hydrobromide according to patent claim 2.

6. Use of the crystalline hydrochloride according to patent claim 1 for the production of pharmaceutical preparations.

7. Use of the crystalline hydrobromide according to patent claim 2 for the production of pharmaceutical preparations.

## Claims for the Contracting State: AT

1. Process for the production of crystalline 7β-[2-(2-amino-4-thiazolyl)-2-methoxyiminoacetamido]-3-cephem-4-carboxylic acid pivaloyloxymethyl ester hydrochloride, characterised in that 7β-[2-(2-amino-4-thiazolyl)-2-methoxyiminoacetamido]-3-cephem-4-carboxylic acid pivaloyloxymethyl ester is converted by treatment with hydrogen chloride into the hydrochloride, and this is caused to crystallise.

2. Process for the production of crystalline 7β-[2-(2-amino-4-thiazolyl)-2-methoxyiminoacetamido]-3-cephem-4-carboxylic acid pivaloyloxymethyl ester hydrobromide, characterised in that 7β-[2-(2-amino-4-thiazolyl)-2-methoxyiminoacetamido]-3-cephem-4-carboxylic acid pivaloyloxymethyl ester is converted by treatment with hydrogen bromide into the hydrobromide, and this is caused to crystallise.

3. Use of the crystalline hydrochloride obtained according to patent claim 1 for the production of pharmaceutical proparations.

4. Use of the crystalline hydrobromide obtained according to patent claim 2 for the production of pharmaceutical preparations.

## Revendications pour les Etats contractants: BE, CH, DE, FR, GB, IT, LI, LU, NL, SE

1. Le chlorhydrate cristallisé du 7β-[2-(2-amino-4-thiazolyl)-2-méthoxyiminoacétamido]-3-céphème-4-carboxylate de pivaloyloxyméthyle.

2. Le bromhydrate cristallisé du 7β-[2-(2-amino-4-thiazolyl)-2-méthoxyiminoacétamido]-3-céphème-4-carboxylate de pivaloyloxyméthyle.

3. Procédé de préparation du chlorhydrate ou du bromhydrate cristallisé du 7β-[2-(2-amino-4-thiazo-lyl)-2-méthoxyiminoacétamido]-3-céphème-4-carboxylate de pivaloyloxyméthyle, caractérisé en ce que l'on convertit le 7β-[2-(2-amino-4-thiazolyl)-2-méthoxyiminoacétamido]-3-céphème-4-carboxylate de pivaloyloxyméthyle en chlorhydrate ou bromhydrate par traitement à l'aide du chlorure d'hydrogène ou du bromure d'hydrogène et on fait cristalliser le chlorhydrate ou bromhydrate.

4. Compositions pharmaceutiques contenant le chlorhydrate cristallisé selon la revendication 1.

5. Compositions pharmaceutiques contenant le bromhydrate cristallisé selon la revendication 2.

6. Utilisation du chlorhydrate cristallisé selon la revendication I pour la préparation de compositions pharmaceutiques.

7. Utilisation du bromhydrate cristallisé selon la revendication 2, pour la préparation de compositions pharmaceutiques.

**Revendications pour l'Etat Contractant: AT**

1. Procédé de préparation du chlorhydrate cristallisé du 7$\beta$-[2-(2-amino-4-thiazolyl)-2-méthoxyiminoacétamido]-3-céphème-4-carboxylate de pivaloyloxyméthyle caractérisé en ce que l'on convertit le 7$\beta$-[2-(2-amino-4-thiazolyl)-2-méthoxyiminoacétamido]-3-céphème-4-carboxylate de pivaloyloxyméthyle en chlorhydrate par traitement à l'aide du chlorure d'hydrogène et on fait cristalliser le chlorhydrate.

2. Procédé de préparation du bromhydrate cristallisé du 7$\beta$-[2-(2-amino-4-thiazolyl)-2-méthoxyiminoacétamido]-3-céphème-4-carboxylate de pivaloyloxométhyle caractérisé en ce que l'on convertit le 7$\beta$-[2-(2-amino-4-thiazolyl)-2-méthoxyiminoacétamido]-3-céphème-4-carboxylate de pivaloyloxyméthyle en bromhydrate par traitement à l'aide du bromure d'hydrogène et on fait cristalliser le bromhydrate.

3. Utilisation du chlorhydrate cristallisé obtenu selon la revendication I pour la préparation de compositions pharmaceutiques.

4. Utilisation du bromhydrate cristallisé obtenu selon la revendication 2 pour la préparation de compositions pharmaceutiques.